# EUROPÄISCHE PATENTANMELDUNG

(11) **EP 4 717 204 A1**
(43) Veröffentlichungstag der Anmeldung: **01.04.2026**
(21) Anmeldenummer: 25202097.9
(22) Anmeldetag: 15.09.2025
(51) Int. Cl.: A61B 17/80

(54) **OSTEOSYNTHESEPLATTE**

(30) Priorität: 25.09.2024 DE 102024127785
(71) Anmelder: Karl Leibinger Asset Management GmbH & Co. KG, 78570 Mühlheim an der Donau (DE)
(72) Erfinder: Pabst, Martin, 78479 Reichenau (DE); Dilger, Pascal, 78601 Mahlstetten (DE); Pfeiffer, Vincent, 78570 Mühlheim (DE); Samyn, Thomas, 78234 Engen-Welschingen (DE); Dr. Diener, Tobias, 78467 Konstanz (DE)
(74) Vertreter: Schwamberger, Martin

(57) **Zusammenfassung**

Osteosyntheseplatte (P) mit einem länglichen Grundkörper (G), welcher eine Oberseite (G1), eine Unterseite (G2) sowie einen Kopfabschnitt (KA) und einen Schaftabschnitt (SA) aufweist. An dem Kopfabschnitt (KA) und an dem Schaftabschnitt (SA) sind mehrere Durchgangsöffnungen (A1 bis A12) angeordnet, die für die Aufnahme von je einer Schraube (SK) vorgesehen sind und zwischen der Oberseite (G1) und der Unterseite (G2) des Grundkörpers (G) entlang je einer zugeordneten Mittelachse (MA1 bis MA12) verlaufen. Dabei sind an dem Kopfabschnitt (KA) vier Durchgangsöffnungspaare (APa1, APa2, APa3, APa4) sowie eine einzelne Durchgangsöffnung (A7) ausgestaltet, wobei ausgehend von einem proximalen Ende (E1) in Längsrichtung des Grundkörpers (G) am Kopfabschnitt (K) zunächst das erste Durchgangsöffnungspaar (APa1), dann das zweite Durchgangsöffnungspaar (APa2), dann das dritte Durchgangsöffnungspaar (APa3), dann die einzelne Durchgangsöffnung (A7) und schließlich das vierte Durchgangsöffnungspaar (APa4) aufeinander folgen.

## Beschreibung

Die Erfindung betrifft eine Osteosyntheseplatte, welche für eine Befestigung am proximalen Humerus zur Fixierung von Knochenfragmenten vorgesehen ist.

Bei der Osteosynthese werden nach einer Knochenfraktur im Rahmen eines operativen Eingriffs zwei oder mehr Knochenfragmente miteinander verbunden, um ein Zusammenwachsen der Knochenfragmente zu erreichen. Ziel der Osteosynthese ist dabei eine stabile Fixierung der Knochenfragmente, wobei diese Fixierung dabei in einer korrekten Stellung der Knochenfragmente zueinander und somit ggf. unter Korrektur von Fehlstellungen erfolgen soll. Neben einer Fixierung über Draht oder Schrauben kommen je nach Anwendungsbereich auch Osteosyntheseplatten zur Anwendung, wobei die jeweilige Osteosyntheseplatte dabei im Bereich der Fraktur auf den jeweiligen Knochen aufgelegt und an den miteinander zu verbindenden Knochenfragmenten jeweils befestigt wird. Eine Befestigung wird dabei zumeist über Schrauben vorgenommen, wozu an der jeweiligen Osteosyntheseplatte dann mehrere Durchgangsöffnungen ausgebildet sind.

Aus der US 2012/0191104 A1 geht eine Osteosyntheseplatte hervor, welche für eine Befestigung am proximalen Humerus vorgesehen ist. Konkret dient die Osteosyntheseplatte der US 2012/0191104 A1 der lateralen Befestigung zur Fixierung von Knochenfragmenten bei einer proximalen Humerusfraktur. Die Osteosyntheseplatte weist dabei einen länglichen Grundkörper auf, dessen Unterseite für eine Auflage der Osteosyntheseplatte auf den miteinander zu verbindenden Knochenfragmenten vorgesehen ist. Zudem sind bei dem Grundkörper ein Kopfabschnitt und ein hieran anschließender Schaftabschnitt definiert, von welchen der Kopfabschnitt einer Auflage im Bereich eines Humeruskopfs und -halses dient, während der Schaftabschnitt für eine Auflage an einem Humerusschaft vorgesehen ist. Sowohl der Kopfabschnitt als auch der Schaftabschnitt weisen mehrere Durchgangsöffnungen auf, die jeweils zwischen der Unterseite des Grundkörpers und einer der Unterseite abgewandt liegenden Oberseite entlang je einer zugehörigen Mittelachse verlaufen. Jede der Durchgangsöffnungen dient dabei der Aufnahme jeweils einer Schraube, über welche die Osteosyntheseplatte in dem entsprechenden Bereich auf ihrer Unterseite an dem dort liegenden Knochenfragment befestigt werden kann. Während die Durchgangsöffnungen am Schaftabschnitt in Längsrichtung des Grundkörpers hintereinanderliegend als Langlöcher gestaltet sind, sind bei dem Kopfabschnitt vier Durchgangsöffnungspaare sowie eine einzelne Durchgangsöffnung ausgestaltet. Von einem proximalen Ende her folgen dabei zunächst drei der Durchgangsöffnungspaare, dann die einzelne Durchgangsöffnung und dann ein weiteres Durchgangsöffnungspaar aufeinander.

Ausgehend vom vorstehend beschriebenen Stand der Technik ist es nun die Aufgabe der vorliegenden Erfindung eine Osteosyntheseplatte zu schaffen, bei welcher eine möglichst zuverlässige Fixierung am proximalen Humerus realisierbar ist.

Diese Aufgabe wird ausgehend vom Oberbegriff des Anspruchs 1 in Verbindung mit dessen kennzeichnenden Merkmalen gelöst. Die hierauf folgenden, abhängigen Ansprüche geben jeweils vorteilhafte Weiterbildungen der Erfindung wieder. Weitere vorteilhafte Ausgestaltungen ergeben sich aus der Beschreibung sowie aus den Figuren.

Gemäß der Erfindung weist eine Osteosyntheseplatte, welche für eine Befestigung am proximalen Humerus zur Fixierung von Knochenfragmenten vorgesehen ist, einen länglichen Grundkörper auf. Dieser längliche Grundkörper ist hierbei mit einer Oberseite und mit einer der Oberseite abgewandt liegenden Unterseite ausgestattet, wobei der Grundkörper zudem über einen Kopfabschnitt und einen Schaftabschnitt verfügt. Die Unterseite ist zur Auflage der Osteosyntheseplatte auf den Knochenfragmenten eingerichtet. An dem Kopfabschnitt und an dem Schaftabschnitt sind jeweils mehrere Durchgangsöffnungen ausgestaltet, die jeweils für die Aufnahme von je einer Schraube vorgesehen sind und jeweils zwischen der Oberseite und der Unterseite des Grundkörpers entlang je einer zugeordneten Mittelachse verlaufen. Dabei sind an dem Kopfabschnitt vier Durchgangsöffnungspaare sowie eine einzelne Durchgangsöffnung ausgestaltet. Ausgehend von einem proximalen Ende folgen am Kopfabschnitt in Längsrichtung des Grundkörpers zunächst das erste Durchgangsöffnungspaar, dann das zweite Durchgangsöffnungspaar, dann das dritte Durchgangsöffnungspaar, dann die einzelne Durchgangsöffnung und schließlich das vierte Durchgangsöffnungspaar aufeinander. Des Weiteren verlaufen die Mittelachsen der Durchgangsöffnungen des ersten Durchgangsöffnungspaares in Längsrichtung des Grundkörpers betrachtet zumindest weitestgehend parallel zueinander.

Die erfindungsgemäße Osteosyntheseplatte ist also für die Fixierung von Knochenfragmenten am proximalen Humerus vorgesehen, wobei die Osteosyntheseplatte dabei bevorzugt einer lateralen Befestigung am proximalen Humerus dient. Für die Fixierung an den Knochenfragmenten wird die erfindungsgemäße Osteosyntheseplatte an einer Unterseite ihres länglichen Grundkörpers auf die miteinander zu verbindenden Knochenfragmente aufgelegt, wobei über die Osteosyntheseplatte dabei insbesondere zwei im Rahmen einer Humerusfraktur gebildeten Knochenfragmente miteinander verbunden werden können.

Bevorzugt folgen der Schaftabschnitt und der Kopfabschnitt in Längsrichtung des Grundkörpers, d.h. entlang einer Längsachse, in deren Richtung der Grundkörper der Osteosyntheseplatte seine größte Erstreckung aufweist, aufeinander. Dabei sind der Schaftabschnitt und der Kopfabschnitt insbesondere in Längsrichtung unmittelbar aneinander angrenzend ausgestaltet. Bevorzugt ist der Kopfabschnitt dabei in Längsrichtung an einem Ende ausgestaltet, welches bei Befestigung der erfindungsgemäßen Osteosyntheseplatte am proximalen Humerus proximal liegt, wohingegen der Schaftabschnitt an einem hierzu entgegengesetzten Ende ausgebildet ist, mit dem die Osteosyntheseplatte bei Befestigung distal liegt. Ganz besonders bevorzugt ist der Kopfabschnitt des Grundkörpers der Osteosyntheseplatte dabei für eine Auflage im Bereich des Humeruskopfs und des -halses vorgesehen, während der Schaftabschnitt des Grundkörpers einer Auflage im Bereich des Humerusschafts dient.

Insbesondere ist der Kopfabschnitt des Grundkörpers in einer Querrichtung, d.h. entlang einer Querachse, welche orthogonal zu der Längsachse in einer Breitenrichtung des Grundkörpers verläuft, mit einer größeren Erstreckung ausgeführt als der Schaftabschnitt.

In den länglichen Grundkörper sind sowohl an dem Kopfabschnitt als auch an dem Schaftabschnitt Durchgangsöffnungen eingebracht, wobei jede dieser Durchgangsöffnungen dabei zwischen der Oberseite und der Unterseite des Grundkörpers verläuft. Dabei findet der jeweilige Verlauf der jeweiligen Durchgangsöffnung durch den Grundkörper zwischen der Ober- und der Unterseite entlang je einer zugehörigen Mittelachse statt. Ferner dient die jeweilige Durchgangsöffnung der Hindurchführung je einer Schraube, über welche die erfindungsgemäße Osteosyntheseplatte mit dem Knochen verbunden wird.

Im Rahmen der Erfindung kann die jeweilige Durchgangsöffnung dabei dazu gestaltet sein, unterschiedliche Schrauben aufzunehmen, wobei hierbei bevorzugt unterschiedliche Arten von Schrauben und/oder unterschiedliche Schaftdurchmesser von Schrauben aufgenommen werden können. Als Arten von Schrauben können dabei bevorzugt zum einen Verriegelungsschrauben sowie zum anderen Kompressionsschrauben durch die jeweilige Durchgangsöffnungen durchgeführt werden. Auf dem Fachmann prinzipiell bekannte Weise ist eine Verriegelungsschraube dabei insbesondere an einem Schraubenkopf mit einem konischen Kopfgewinde ausgestattet und weist an einem an den Schraubenkopf anschließenden Schraubenschaft ein Schaftgewinde auf. Hingegen ist bei einer Kompressionsschraube ein kugelförmiger Schraubenkopf vorgesehen, an welchen sich ein Schraubenschaft mit einem Schaftgewinde anschließt. Sowohl eine Verriegelungsschraube als auch eine Kompressionsschraube kann dabei prinzipiell als Kortikalisschraube oder als Spongiosaschraube mit Teilgewinde oder Vollgewinde ausgeführt sein. Ganz besonders bevorzugt können bei den Durchgangsöffnungen des Kopfabschnitts dabei jeweils zum einen Verriegelungsschrauben, sowie zum anderen Kompressionsschrauben aufgenommen werden.

Bei der erfindungsgemäßen Osteosyntheseplatte sind an dem Kopfabschnitt des Grundkörpers vier Durchgangsöffnungspaare sowie eine einzelne Durchgangsöffnung ausgestaltet. Dabei liegen die jeweils zugehörigen Durchgangsöffnungen eines jeweiligen Durchgangsöffnungspaares des Kopfabschnitts bevorzugt beidseitig exzentrisch zu einer Längsmittelebene, welche den Grundkörper in seiner Längsrichtung zentrisch durchläuft und dabei Oberseite und Unterseite jeweils durchdringt. Hierbei liegen die jeweiligen Durchgangsöffnungen des jeweiligen Durchgangsöffnungspaare jeweils bevorzugt überwiegend oder vollständig auf je einer Seite der Längsmittelebene, d.h. sie sind jeweils nur zu einem geringen Teil oder gar nicht von der Längsmittelebene durchdrungen. Des Weiteren überdecken sich die einander zugeordneten Durchgangsöffnungen des jeweiligen Durchgangsöffnungspaares insbesondere in Längsrichtung des Grundkörpers miteinander, wobei sich die einander zugeordneten Durchgangsöffnungen des jeweiligen Durchgangsöffnungspaares dabei in Längsrichtung vollständig, überwiegend oder mit einem geringeren Anteil überdecken können.

Hingegen liegt die einzelne Durchgangsöffnung des Kopfabschnitts bevorzugt in der Längsmittelebene, wobei die einzelne Durchgangsöffnung dabei zentrisch oder exzentrisch zu der Längsmittelebene ausgestaltet sein kann. Ganz besonders bevorzugt liegt die einzelne Durchgangsöffnung des Kopfabschnitt exzentrisch zu der Längsmittelebene und ist nur zu einem geringen Teil von der Längsmittelebene durchdrungen.

Von einem proximalen Ende des Grundkörpers, welches aufgrund seiner Lage bei Anordnung der Osteosyntheseplatte am proximalen Humerus auch als craniales Ende bezeichnet sein kann, ist an dem Kopfabschnitt in Längsrichtung des Grundkörpers zunächst das erste Durchgangsöffnungspaar, dann das zweite Durchgangsöffnungspaar, dann das dritte Durchgangsöffnungspaar, dann die einzelne Durchgangsöffnung und schließlich das vierte Durchgangsöffnungspaar vorgesehen. Bei dem ersten Durchgangsöffnungspaar verlaufen die Mittelachsen der zugehörigen Durchgangsöffnungen in Längsrichtung des Grundkörpers betrachtet zumindest weitestgehend parallel zueinander.

Die Erfindung umfasst nun die technische Lehre, dass die Mittelachsen der Durchgangsöffnungen des zweiten Durchgangsöffnungspaares in Längsrichtung des Grundkörpers betrachtet auf der Unterseite kreuzend zueinander verlaufen. Ferner verlaufen die Mittelachsen der Durchgangsöffnungen des dritten Durchgangsöffnungspaares in Längsrichtung des Grundkörpers betrachtet auf der Unterseite divergierend zueinander, wohingegen die Mittelachsen der Durchgangsöffnungen des vierten Durchgangsöffnungspaares in Längsrichtung des Grundkörpers betrachtet zumindest weitestgehend parallel zueinander verlaufen.

An dem Kopfabschnitt des Grundkörpers findet also in Längsrichtung des Grundkörpers betrachtet bei den Durchgangsöffnungen des ersten Durchgangsöffnungspaares zumindest im Wesentlichen ein paralleler Verlauf der Mittelachsen statt. Hingegen kreuzen sich die Mittelachsen der Durchgangsöffnungen des zweiten Durchgangsöffnungspaares in Längsrichtung des Grundkörpers betrachtet auf der Unterseite. Die Mittelachsen der Durchgangsöffnungen des dritten Durchgangsöffnungspaares laufen in Längsrichtung des Grundkörpers betrachtet auf der Unterseite auseinander. Bei den Durchgangsöffnungen des vierten Durchgangsöffnungspaares ist in Längsrichtung des Grundkörpers betrachtet erneut ein im Wesentlichen paralleler Verlauf der Mittelachsen realisiert.

Eine derartige Ausgestaltung einer Osteosyntheseplatte hat dabei den Vorteil, dass durch diese in Längsrichtung des Grundkörpers betrachtete Ausrichtung der Mittelachsen eine breit aufgefächerte Befestigung der Osteosyntheseplatte am Kopfabschnitt des Grundkörpers seitens des zugehörigen Knochenfragments möglich, und dadurch auch eine zuverlässige Fixierung realisierbar ist. Denn diese Ausrichtung der Mittelachsen hat sich insbesondere im Hinblick auf die Fixierung einer Osteosyntheseplatte im Bereich des Humeruskopfes und halses als besonders vorteilhaft erwiesen, da aufgrund der gewählten Verläufe der Mittelachsen ein breites Auffächern der Schraubenverläufe stattfindet.

Im Rahmen der Erfindung ist unter einem "zumindest überwiegend" oder "zumindest weitestgehend" parallelen Verlauf neben einem absolut parallelen Verlauf auch eine Winkelabweichung von einer Parallelität um einige wenige Grad bis maximal 12° zu verstehen.

Gemäß einer vorteilhaften Ausgestaltungsmöglichkeit der Erfindung fällt eine am proximalen Ende liegende Kante des Kopfabschnitts zu einer Seite des Grundkörpers hin ab. Ganz besonders bevorzugt ist dieses Abfallen dabei zu einer posterior liegenden Seite des Grundkörpers vorgenommen, wodurch am proximalen Ende der Osteosyntheseplatte ein ausreichender Freiraum der Osteosyntheseplatte zu dem Schulterblatt bei Bewegungen des Humerus geschaffen ist. Insbesondere fällt die Kante gegenüber einer Querebene des Grundkörpers um 10° bis 20°, bevorzugt um 13 bis 17° sowie besonders bevorzugt um 15° ab. Die Querebene verläuft dabei insbesondere orthogonal zu der Längsmittelebene des Grundkörpers und durchläuft hierbei den Grundkörper in seiner Querrichtung unter Durchdringung der Oberseite und der Unterseite.

Entsprechend einer Ausführungsform der Erfindung sind die jeweils zugehörigen Durchgangsöffnungen des jeweiligen Durchgangsöffnungspaares in Längsrichtung des Grundkörpers versetzt zueinander angeordnet. Dieser Versatz hat sich in Hinblick auf eine erreichbare Steifigkeit bei Befestigung der Osteosyntheseplatte als besonders vorteilhaft erwiesen.

In Weiterbildung der vorgenannten Ausführungsform ist bei dem ersten Durchgangsöffnungspaar, dem zweiten Durchgangsöffnungspaar und dem dritten Durchgangsöffnungspaar die jeweils auf einer posterioren Seite des Grundkörpers liegende Durchgangsöffnung in Längsrichtung distal versetzt zu der jeweils auf einer anterioren Seite des Grundkörpers liegenden Durchgangsöffnung ausgestaltet. Diese Variante der Erfindung ist dabei insbesondere in Kombination mit dem abfallenden Verlauf der am proximalen Ende liegenden Kante vorteilhaft, wobei der jeweilige Versatz der jeweils zugehörigen Durchgangsöffnungen des ersten, des zweiten und des dritten Durchgangsöffnungspaares dabei jeweils zumindest weitestgehend parallel zu der Kante vorgenommen ist.

Alternativ, bevorzugt aber ergänzend zu der vorgenannten Weiterbildung ist bei dem vierten Durchgangsöffnungspaar die auf einer posterioren Seite des Grundkörpers liegende Durchgangsöffnung in Längsrichtung proximal versetzt zu der jeweils auf einer anterioren Seite des Grundkörpers liegenden Durchgangsöffnung ausgestaltet. Dies hat insbesondere in Kombination mit der versetzten Anordnung bei dem dritten Durchgangsöffnungspaar und aufgrund der zwischenliegenden Anordnung der einzelnen Durchgangsöffnung eine entsprechende Verdichtung der Durchgangsöffnungen und damit auch der Befestigungsmöglichkeiten der Osteosyntheseplatte in diesem Bereich zur Folge.

Es ist eine weitere Ausführungsform der Erfindung, dass die Mittelachse der einzelnen Durchgangsöffnung des Kopfabschnitts in Längsrichtung des Grundkörpers betrachtet zumindest weitestgehend orthogonal zu dem dortigen Bereich des Kopfabschnitts verläuft. Insofern steht die Mittelachse der einzelnen Durchgangsöffnung im Bereich der Ausgestaltung der einzelnen Durchgangsöffnung und in Längsrichtung des Grundkörpers betrachtet zumindest im Wesentlichen orthogonal zu dem Kopfabschnitt. Im Rahmen der Erfindung ist unter einem "zumindest überwiegend" oder "zumindest weitestgehend" orthogonalen Verlauf neben einem absolut rechtwinkligen Verlauf auch eine Winkelabweichung von einer Orthogonalität um einige wenige Grad bis maximal 10° zu verstehen.

In Weiterbildung der Erfindung folgen der Kopfabschnitt und der Schaftabschnitt entlang einer Längsmittelebene des Grundkörpers geradlinig aufeinander. Insofern sind der Kopfabschnitt und der Schaftabschnitt des Grundkörpers in Längsrichtung in einer Linie angeordnet, also in Längsrichtung nicht gegeneinander anguliert.

Gemäß einer weiteren Ausführungsform der Erfindung ist der Kopfabschnitt in Längsrichtung des Grundkörpers betrachtet gegenüber dem Schaftabschnitt zu einer anterioren Seite des Grundkörpers hin geneigt verdreht. Ganz besonders bevorzugt ist diese Verdrehung um 4° bis 10° vorgenommen.

Es ist eine vorteilhafte Ausgestaltungsmöglichkeit der Erfindung, dass die Mittelachsen der Durchgangsöffnungen des ersten Durchgangsöffnungspaares, die Mittelachsen der Durchgangsöffnungen des zweiten Durchgangsöffnungspaares, die Mittelachsen der Durchgangsöffnungen des dritten Durchgangsöffnungspaares, die Mittelachse der einzelnen Durchgangsöffnung sowie die Mittelachsen der Durchgangsöffnungen des vierten Durchgangsöffnungspaares in Querrichtung des Grundkörpers betrachtet auf der Unterseite jeweils in Richtung des proximalen Endes verkippt sind. In vorteilhafter Weise können hierdurch in Querrichtung des Grundkörpers betrachtet beim Kopfabschnitt jeweils Verläufe der Mittelachsen verwirklicht werden, bei welchen die hindurchgeführten Schrauben jeweils den Bereich des Humeruskopfs und -halses treffen können. Besonders bevorzugt verlaufen die Mittelachsen der Durchgangsöffnungen des vierten Durchgangsöffnungspaares in Querrichtung des Grundkörpers betrachtet dabei so, dass die Mittelachsen zumindest der einzelnen Durchgangsöffnung und der Durchgangsöffnungen des dritten Durchgangsöffnungspaares auf Einschraubtiefe der zugehörigen Schrauben geschnitten werden. Insofern ist hinsichtlich der Mittelachsen der Durchgangsöffnungen des vierten Durchgangsöffnungspaares ein proximal stärkerer Anstieg auf der Unterseite zu realisieren.

Besonders bevorzugt verläuft der Schaftabschnitt zumindest weitestgehend eben. Dabei definiert der Schaftabschnitt eine Längsmittelebene des Grundkörpers und eine orthogonal zu der Längsmittelebene verlaufende Querebene des Grundkörpers. Insbesondere durchläuft die Längsmittelebene den Grundkörper in seiner Längsrichtung zentrisch und durchdringt dabei Oberseite und Unterseite, wobei auch die orthogonal hierzu verlaufende Querebene Oberseite und Unterseite durchdringt. Weiter bevorzugt definiert der zumindest weitestgehend eben verlaufende Schaftabschnitt auch eine Tiefenebene, welche orthogonal zu der Längsmittelebene und der Querebene ausgerichtet ist.

Hinsichtlich dieser definierten Ebenen sind die Mittelachsen der Durchgangsöffnungen des Kopfabschnitts dann derartig ausgerichtet, dass
- die Mittelachsen der Durchgangsöffnungen des ersten Durchgangsöffnungspaares gegenüber der Querebene auf der Unterseite in Richtung des proximalen Endes in einem Winkel im Bereich 10° bis 25° ausgerichtet sind, und/oder
- die Mittelachsen der Durchgangsöffnungen des zweiten Durchgangsöffnungspaares in Längsrichtung des Grundkörpers betrachtet auf der Unterseite unter einem Winkel im Bereich 40° bis 53° kreuzend ausgerichtet sind, und/oder
- die Mittelachsen der Durchgangsöffnungen des zweiten Durchgangsöffnungspaares gegenüber der Querebene auf der Unterseite in Richtung des proximalen Endes in einem Winkel im Bereich 5° bis 20° ausgerichtet sind, und/oder
- die Mittelachsen der Durchgangsöffnungen des dritten Durchgangsöffnungspaares in Längsrichtung des Grundkörpers betrachtet auf der Unterseite (G2) unter einem Winkel im Bereich 15° bis 30° divergierend ausgerichtet sind, und/oder
- die Mittelachsen der Durchgangsöffnungen des dritten Durchgangsöffnungspaares gegenüber der Querebene auf der Unterseite in Richtung des proximalen Endes in einem Winkel im Bereich 5° bis 16° ausgerichtet sind, und/oder
- die Mittelachse der einzelnen Durchgangsöffnung gegenüber der Querebene auf der Unterseite in Richtung des proximalen Endes in einem Winkel im Bereich 10° bis 16° ausgerichtet ist, und/oder
- die Mittelachsen der Durchgangsöffnungen des vierten Durchgangsöffnungspaares in Richtung des proximalen Endes in einem Winkel im Bereich 20° bis 35° ausgerichtet sind.

Diese Verläufe haben sich hinsichtlich des Kopfabschnitt der Osteosyntheseplatte als besonders vorteilhaft erwiesen.

Entsprechend einer weiteren Ausgestaltungsmöglichkeit der Erfindung sind an dem Schaftabschnitt eine erste Durchgangsöffnung, eine zweite Durchgangsöffnung und eine dritte Durchgangsöffnung ausgestaltet, die in Längsrichtung des Schaftabschnitts hintereinanderliegend angeordnet sind sowie jeweils eine Längsmittelebene des Grundkörpers schneiden. Dies hat den Vorteil, dass hierdurch auch seitens des Schaftabschnitts geeignete Durchgangsöffnungen zur Befestigung an einem Knochenfragment geschaffen werden. Insbesondere ist die benachbart zu dem Kopfabschnitt liegende, erste Durchgangsöffnung als Langloch ausgeführt, wodurch eine Position der durch die erste Durchgangsöffnung hindurchgeführten Schraube in Längsrichtung variiert werden kann. Dazu ist das Langloch insbesondere in Längsrichtung des Grundkörpers verlaufend orientiert. Die als Langloch ausgeführte, erste Durchgangsöffnung ist dabei bevorzugt dazu geeignet, eine Kompressionsschraube aufzunehmen. Zudem wird bei Ausgestaltung der ersten Durchgangsöffnung als Langloch bei der ersten Durchgangsöffnung ein Bereich definiert, in welchem eine Mittelachse der ersten Durchgangsöffnung liegt.

Entsprechend einer weiteren Ausführungsform der Erfindung lassen eine oder mehrere der Durchgangsöffnungen jeweils ein Verschwenken der je einen Schraube gegenüber der jeweiligen Mittelachse zu, bevorzugt im Bereich einer Kegelmantelfläche, besonders bevorzugt einer Kegelmantelfläche mit einem Öffnungswinkel von 30°. Insbesondere ist die jeweilige das Verschwenken zulassende Durchgangsöffnung dazu mit einem Einsatz versehen, wobei dieser Einsatz dabei im Vergleich zu dem restlichen Grundkörper aus einem Material mit höherer Duktilität besteht, wie vorzugsweise Reintitan. Hingegen ist der Grundkörper bevorzugt aus einer Titanlegierung oder Stahl hergestellt.

Im Rahmen der Erfindung kann der Grundkörper in seinem Randbereich mit als Nahtanker gestalteten Fadenlöchern ausgestattet sein, welche jeweils einem Hindurchführen von Fäden zum Anbinden von Gewebe an der Osteosyntheseplatte dienen. Insbesondere besitzen diese Fadenlöcher dabei auf der Unterseite des Grundkörpers jeweils je einen Unterschnitt.

Alternativ oder ergänzend zu den vorgenannten Varianten können auf der Unterseite oder der Oberseite des Grundkörpers oder auch auf beiden Seiten Vertiefungen eingefräst sein, wobei diese Vertiefungen dabei zwischen den Durchgangsöffnungen ausgestaltet sein können.

Weiter alternativ oder ergänzend können in den Grundkörper weitere Durchbrüche eingebracht sein, die jeweils der Durchführung eines Befestigungsdrahtes oder ähnlichem dienen. Zudem kann auf dem Grundkörper der Osteosyntheseplatte eine Befestigungsmöglichkeit für eine Bohrplatte ausgestaltet sein, über welche dem Chirurgen beim Bohren von Aufnahmebohrungen für die Schrauben eine Hilfestellung zur Ausrichtung der Bohrungen gegeben wird. Diese Befestigungsmöglichkeit kann dabei beispielsweise in Form einer oder mehrerer Blindbohrungen im Grundkörper vorliegen.

Eine vorteilhafte Ausführungsform der Erfindung, die nachfolgend erläutert wird, ist in den Zeichnungen dargestellt. Es zeigen:
- Fig. 1 bis 3: unterschiedliche Ansichten einer Osteosyntheseplatte gemäß einer bevorzugten Ausführungsform der Erfindung;
- Fig. 4: eine Draufsicht der Osteosyntheseplatte aus den Fig. 1 bis 3, gezeigt mit eingefügten Schrauben;
- Fig. 5 bis 12: jeweils Ansichten der Osteosyntheseplatte gemäß der Fig. 1 bis 3, jeweils betrachtet einerseits in Längsrichtung sowie andererseits in Querrichtung und jeweils unter Darstellung von Verläufen einzelner Mittelachsen von Aufnahmeöffnungen der Osteosyntheseplatte;
- Fig. 13 und 14: Darstellungen eines proximalen Humerus mit der hieran befestigten Osteosyntheseplatte; und
- Fig. 15: eine Ansicht der Unterseite der Osteosyntheseplatte, gezeigt mit eingefügten Schrauben.

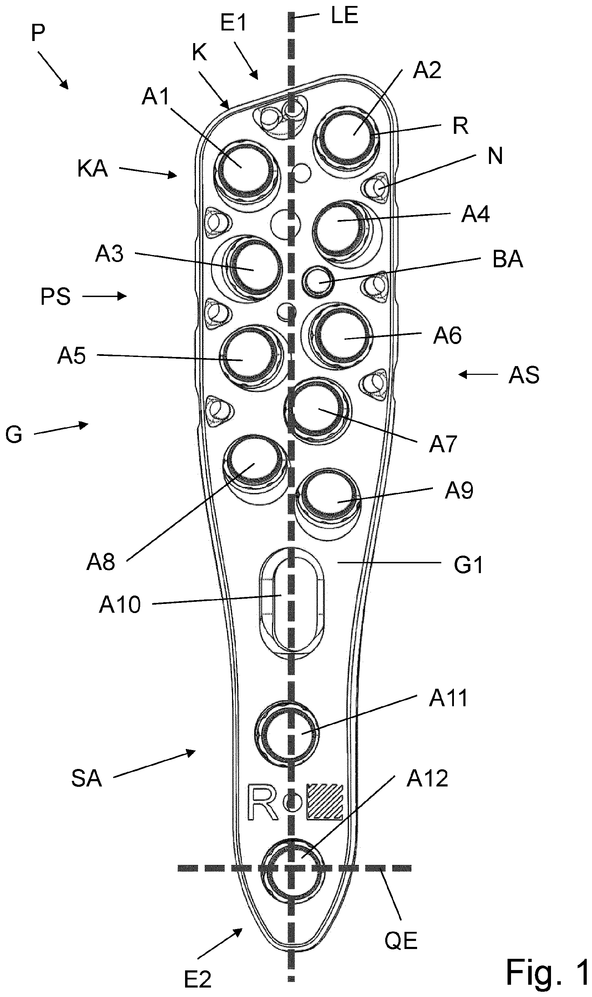

Aus den Fig. 1 bis 15 gehen unterschiedliche Ansichten einer Osteosyntheseplatte P hervor, welche dabei entsprechend einer bevorzugten Ausführungsform der Erfindung ausgestaltet ist. Die Osteosyntheseplatte P weist dabei einen länglichen Grundkörper G auf, der mit einer Oberseite G1 und einer der Oberseite G1 abgewandt liegenden Unterseite G2 versehen ist. Außerdem verfügt der Grundkörper G der Osteosyntheseplatte P über einen Kopfabschnitt KA und einen Schaftabschnitt SA. Insbesondere anhand von Fig. 1 ist zu erkennen, dass der Kopfabschnitt KA und der Schaftabschnitt SA in einer Längsrichtung des Grundkörpers G geradlinig aufeinanderfolgen. In Breitenrichtung weist der Kopfabschnitt KA zudem im Vergleich zum Schaftabschnitt SA eine größere Erstreckung auf.

Die Osteosyntheseplatte P ist für eine laterale Befestigung am proximalen Humerus vorgesehen und dient dabei der Fixierung von Knochenfragmenten, welche aufgrund einer proximalen Humerusfraktur vorliegen. Dabei erfolgt eine Auflage der Osteosyntheseplatte P auf den Knochenfragmenten an der Unterseite G2 des Grundkörpers G, wobei der Kopfabschnitt KA an einem proximal liegenden Ende E1 des Grundkörpers G liegt und hierbei insbesondere für eine Befestigung im Bereich des Humeruskopfes und des -halses vorgesehen ist, während der Schaftabschnitt SA an einem entgegengesetzt zu dem proximalen Ende E1 liegenden, distalen Ende E2 vorgesehen ist und bevorzugt einer Befestigung im Bereich des Humerusschafts dient.

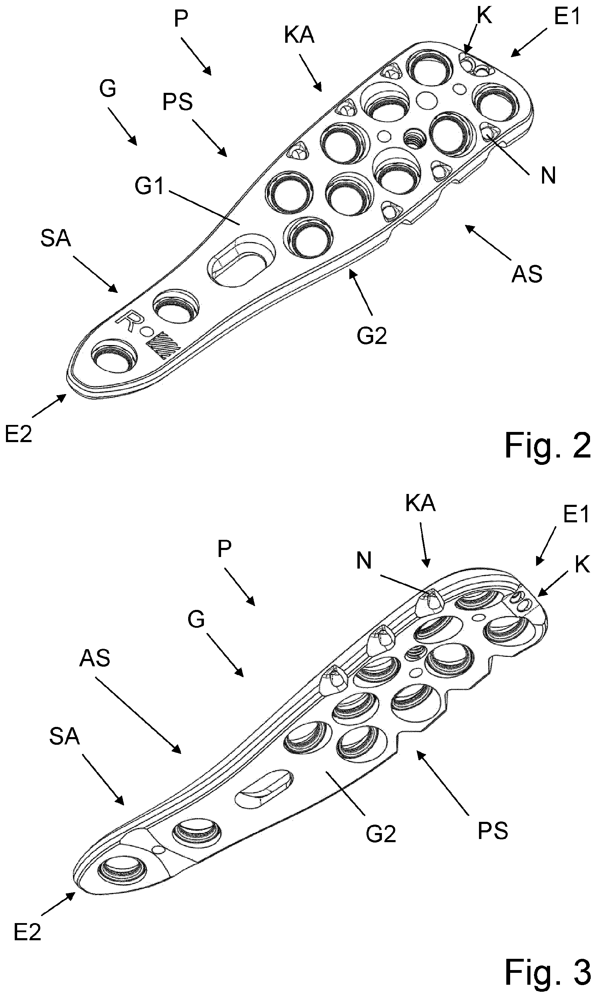

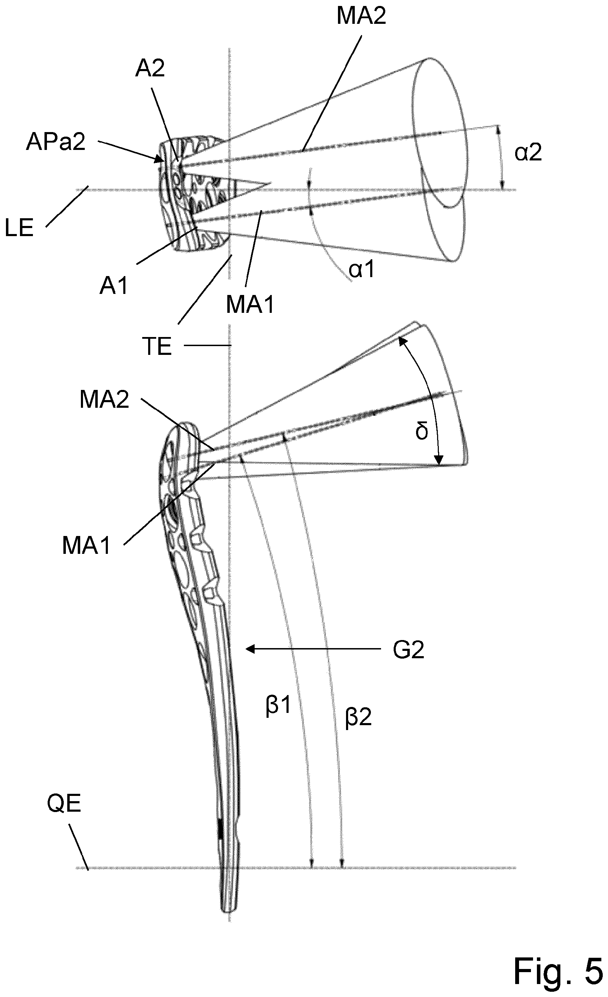

Der Schaftabschnitt SA des Grundkörpers G verläuft zumindest weitestgehend eben und definiert eine Längsmittelebene LE, die in Längsrichtung des Grundkörpers G zentrisch verläuft und die Oberseite G1 und die Unterseite G2 jeweils durchdringt, eine Querebene QE, welche in Querrichtung des Grundkörpers G am Schaftabschnitt SA orthogonal zu der Längsmittelebene LE verläuft und ebenfalls die Oberseite G1 und die Unterseite G2 jeweils durchdringt, sowie eine - in den Fig. 5 bis 12 jeweils angedeutete und dort jeweils in die jeweilige Zeichenebene hineinlaufende - Tiefenebene TE, die sowohl zu der Längsmittelebene LE, als auch zu der Querebene QE jeweils orthogonal ausgerichtet ist und in dem Schaftabschnitt SA in Tiefenrichtung mittig zwischen der Oberseite G1 und der Unterseite G2 verläuft. Ausgehend von dem Schaftabschnitt SA weist der Kopfabschnitt KA eine leichte Kröpfung in Richtung der Oberseite G1 auf, wie anhand von Fig. 3 und insbesondere auch anhand von Fig. 5 jeweils zu erkennen ist. Diese Kröpfung ist dabei gewählt, um bei Anlage des Schaftabschnitts SA im Bereich des Humerusschafts die Anlage des Kopfabschnitts KA im Bereich des Humeruskopfs und -halses zu ermöglichen.

Vorliegend ist die Osteosyntheseplatte P für die laterale Befestigung am rechten Humerus ausgestaltet, wodurch der Grundkörper G neben den beiden Enden E1 und E2 auch eine anteriore Seite AS und eine posteriore Seite PS aufweist. Eine für eine laterale Befestigung am linken Humerus ausgebildete Osteosyntheseplatte wird durch Spiegelung des Aufbaus an der Längsmittelebene LE erhalten. Eine Kante K, mit welcher der Kopfabschnitt KA des Grundkörpers G am proximalen Ende E1 versehen ist, ist dabei zu der posterioren Seite PS abfallend ausgebildet, wobei dies dabei gegenüber der Querebene QE um 10° bis 20°, bevorzugt um 13 bis 17° sowie besonders bevorzugt um 15° vorgenommen ist. Außerdem ist der Kopfabschnitt KA gegenüber dem Schaftabschnitt SA zu der anterioren Seite AS des Grundkörpers G hin geneigt verdreht, wobei diese Verdrehung dabei um 4° bis 10° vorgenommen ist. Dies kann dabei insbesondere in den Fig. 2, 3 und 5 bis 12 erkannt werden.

An dem Kopfabschnitt KA des Grundkörpers G sind an den beiden Seiten AS und PS sowie auch an der Kante K mehrere Nahtanker N ausgestaltet, die als Fadenlöcher jeweils der Hindurchführung je eines Fadens zur Befestigung von Gewebe an der Osteosyntheseplatte P dienen und an der Unterseite G2 jeweils mit je einem Unterschnitt ausgeführt sind.

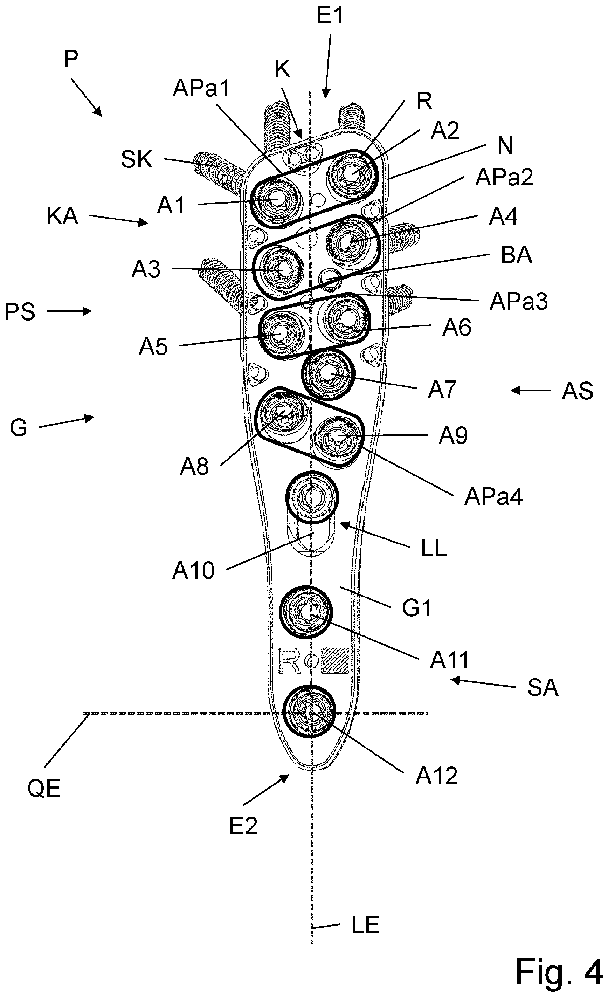

Sowohl an dem Kopfabschnitt KA als auch an dem Schaftabschnitt SA des Grundkörpers G sind mehrere Durchgangsöffnungen A1 bis A12 ausgebildet, die jeweils zwischen der Oberseite G1 und der Unterseite G2 verlaufen und, wie in Fig. 4 gezeigt, für eine Hindurchführung je einer Schraube SK vorgesehen sind. Bei dem Kopfabschnitt KA liegen die zugehörigen Durchgangsöffnungen A1 bis A9 dabei exzentrisch zu der Längsmittelebene LE, wobei beidseitig der Längsmittelebene LE liegende Durchgangsöffnungen A1 bis A6 sowie A8 und A9 dabei paarweise Durchgangsöffnungspaare APa1, APa2, APa3 und APa4 bilden, wie in Fig. 4 angedeutet. Hierbei bilden die Durchgangsöffnungen A1 und A2 das erste Durchgangsöffnungspaar APa1, die Durchgangsöffnungen A3 und A4 das zweite Durchgangsöffnungspaar APa2, die Durchgangsöffnungen A5 und A6 das dritte Durchgangsöffnungspaar APa3 sowie die Durchgangsöffnungen A8 und A9 das vierte Durchgangsöffnungspaar APa4, während die Durchgangsöffnung A7 als einzelne Durchgangsöffnung vorliegt.

Wie anhand von Fig. 4 nachzuvollziehen ist, folgen in Längsrichtung des Grundkörpers G auf das proximalen Ende E1 bei dem Kopfabschnitt KA zunächst das erste Durchgangsöffnungspaar APa1, dann das zweite Durchgangsöffnungspaar APa2, dann das dritte Durchgangsöffnungspaar APa3, dann die einzelne Durchgangsöffnung A7 und schließlich das vierte Durchgangsöffnungspaar APa4. Dabei ist bei den Durchgangsöffnungspaaren APa1, APa2 und APa3 die jeweils auf der posterioren Seite PS liegende Durchgangsöffnung A1 bzw. A3 bzw. A5 in Längsrichtung distal versetzt zu der jeweils auf der anterioren Seite AS liegenden Durchgangsöffnung A2 bzw. A4 bzw. A6 angeordnet. Dagegen liegt die auf der posterioren Seite PS angeordnete Durchgangsöffnung A8 bei dem vierten Durchgangsöffnungspaar APa4 in Längsrichtung proximal versetzt zu der auf der anterioren Seite AS liegenden Durchgangsöffnung A9.

Die Durchgangsöffnungen A1 bis A9 des Kopfabschnitts KA sind dazu geeignet, sowohl Verriegelungsschrauben als auch Kompressionsschrauben aufzunehmen. Dabei ist in der jeweiligen Durchgangsöffnung A1 bzw. A2 bzw. A3 bzw. A4 bzw. A5 bzw. A6 bzw. A7 bzw. A8 bzw. A9 je ein ringförmiger Einsatz R aufgenommen, welcher jeweils im Vergleich zum Grundkörper G aus einem Material mit höherer Duktilität besteht. So sind die Einsätze R bevorzugt aus Reintitan gestaltet, während der Grundkörper G der Osteosyntheseplatte P aus einer Titanlegierung oder Stahl besteht.

Bei dem Schaftabschnitt SA folgen die Durchgangsöffnungen A10 bis A12 in Längsrichtung des Grundkörpers G aufeinander, wobei die Durchgangsöffnungen A10 bis A12 dabei jeweils zumindest annähernd zentrisch in der Längsmittelebene LE liegen. Hierbei ist die Durchgangsöffnung A10 als Langloch LL gestaltet und insbesondere für die Aufnahme einer Kompressionsschraube vorgesehen, während die beiden Durchgangsöffnungen A11 und A12 jeweils dazu geeignet sind, zum einen je eine Verriegelungsschraube sowie zum anderen je eine Kompressionsschraube aufzunehmen. Zudem sind die beiden Durchgangsöffnungen A11 und A12 mit jeweils einem ringförmigen Einsatz R ausgestattet.

Des Weiteren ist der Grundkörper G der Osteosyntheseplatte P noch mit einer Bohrung BA versehen, welche von der Oberseite G1 her in den Kopfabschnitt KA eingebracht und mit einem Innengewinde versehen ist. Über das Innengewinde kann dabei ein nicht dargestellter Bohrführungsblock am Grundkörper G befestigt werden.

Die Durchgangsöffnungen A1 bis A12 sind bei der Osteosyntheseplatte P nun so ausgerichtet, dass bei Befestigung der Osteosyntheseplatte P an den Knochenfragmenten insbesondere im Bereich des Humeruskopfes bzw. -halses eine möglichst große Auffächerung der Schrauben ermöglicht wird, und somit eine zuverlässige Befestigung verbessert. Diese Ausrichtungen werden nun im Folgenden anhand der Fig. 5 bis 12 für die einzelnen Durchgangsöffnungspaare APa1 bis APa4 bzw. für die einzelnen Durchgangsöffnungen A7, A10, A11 und A12 jeweils beschrieben. Dabei ist in den Fig. 5 bis 12 jeweils in der jeweiligen oberen Darstellung je eine Betrachtung des Grundkörpers G in Längsrichtung sowie in der jeweiligen unteren Darstellung je eine Betrachtung des Grundkörpers G in Querrichtung gezeigt.

In Fig. 5 sind Mittelachsen MA1 und MA2 dargestellt, entlang welcher die Durchgangsöffnungen A1 und A2 des Durchgangsöffnungspaares APa1 verlaufen. Dabei ist zu erkennen, dass die beiden Mittelachsen MA1 und MA2 der beiden Durchgangsöffnungen A1 und A2 in Längsrichtung betrachtet (obere Darstellung in Fig. 5) zumindest weitestgehend parallel zueinander verlaufen, wobei die beiden Mittelachsen MA1 und MA2 dabei gegenüber der Längsmittelebene LE jeweils um einen Winkel α1 bzw. α2 zu der posterioren Seite PS des Grundkörpers G hin verkippt sind. Zudem sind die beiden Mittelachsen MA1 und MA2 auf der Unterseite G2 des Grundkörpers G in Querrichtung des Grundkörpers G betrachtet (untere Darstellung in Fig. 5) proximal ansteigend verkippt, wobei hierbei die Mittelachse MA1 der Durchgangsöffnung A1 und die Mittelachse MA2 der Durchgangsöffnung A2 eine proximalen Verkippung mit einem Winkel β1, β2 im Bereich 10 bis 25° aufweist. Über die jeweils aufgenommenen ringförmigen Einsätze R kann die jeweilige Schraube SK zudem gegenüber der jeweiligen Mittelachse MA1 bzw. MA2 innerhalb einer Kegelmantelfläche ausgelenkt werden, welche in Fig. 5 jeweils angedeutet ist und je einen Öffnungswinkel δ von 30° zur Unterseite G2 aufweist.

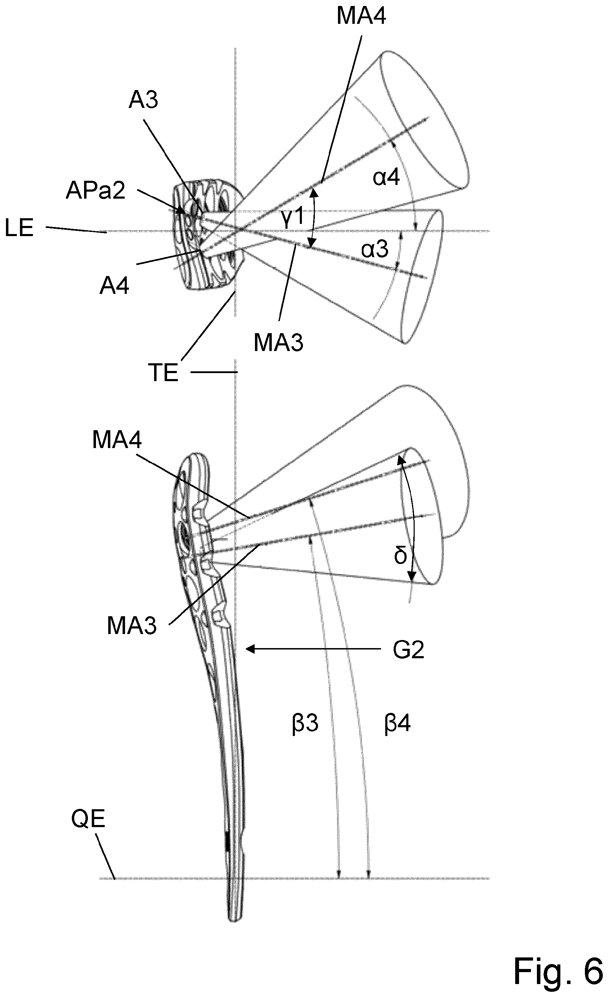

Fig. 6 zeigt die Mittelachsen MA3 und MA4 der Durchgangsöffnungen A3 und A4 des Durchgangsöffnungspaares APa2. Wie dabei zu erkennen ist, verlaufen die beiden Mittelachsen MA3 und MA4 der Durchgangsöffnungen A3 und A4 in Längsrichtung des Grundkörpers G betrachtet (obere Darstellung in Fig. 6) kreuzend auf der Unterseite G2, wobei dieses Kreuzen dabei unter einem Winkel γ1 im Bereich 40° bis 53° stattfindet. Die Mittelachse MA3 der Durchgangsöffnung A3 ist gegenüber der Längsmittelebene LE unter einem Winkel α3 zu der anterioren Seite AS verkippt, wohingegen die Mittelachse MA4 der Durchgangsöffnung A4 gegenüber der Längsmittelebene LE unter einem Winkel α4 zu der posterioren Seite PS verkippt ist.

In Querrichtung des Grundkörpers G betrachtet (untere Darstellung in Fig. 6) sind die beiden Mittelachsen MA3 und MA4 jeweils proximal verkippt. Dabei sind die Mittelachse MA3 der Durchgangsöffnung A3 und die Mittelachse A4 der Durchgangsöffnung A4 unter einem Winkel β3, β4 im Bereich 5° bis 20° proximal verkippt. Erneut können die Schrauben SK in der jeweiligen Durchgangsöffnung A3 bzw. A4 über den jeweiligen Einsatz R innerhalb einer jeweiligen Kegelmantelflächen ausgelenkt werden, wie in Fig. 6 angedeutet. Die jeweilige Kegelmantelflächen verfügt dabei über einen Öffnungswinkel δ von 30° zur Unterseite G2.

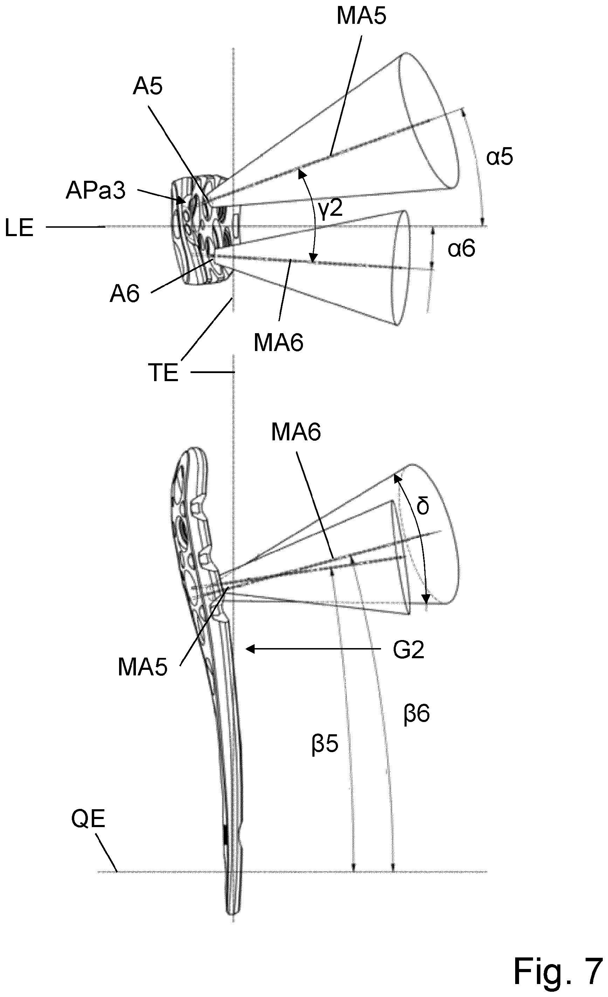

Aus Fig. 7 sind Mittelachsen MA5 und MA6 der Durchgangsöffnungen A5 und A6 des Durchgangsöffnungspaares APa3 ersichtlich. Dabei ist zu erkennen, dass die beiden Mittelachsen MA5 und MA6 der beiden Durchgangsöffnungen A5 und A6 in Längsrichtung betrachtet (obere Darstellung in Fig. 7) auf der Unterseite G2 divergierend zueinander verlaufen, wobei dies dabei mit einem Winkel γ2 im Bereich 15 bis 30° vorgenommen ist. Die Mittelachse MA5 der Durchgangsöffnung A5 ist gegenüber der Längsmittelebene LE unter einem Winkel α5 zu der posterioren Seite PS hin verkippt. Die Mittelachse MA6 der Durchgangsöffnung A6 ist gegenüber der Längsmittelebene LE unter einem Winkel α6 zu der anterioren Seite AS verkippt.

Zudem sind die beiden Mittelachsen MA5 und MA6 auf der Unterseite G2 des Grundkörpers G in Querrichtung des Grundkörpers G betrachtet (untere Darstellung in Fig. 7) proximal ansteigend verkippt. Dabei ist die Mittelachse MA5 der Durchgangsöffnung A5 und die Mittelachse MA6 der Durchgangsöffnung A6 gegenüber der Querebene QE unter einem Winkel β5, β6 im Bereich 5° bis 16° proximal verkippt. In Fig. 7 sind Kegelmantelflächen angedeutet, innerhalb welchen die jeweilige Schraube SK aufgrund des jeweiligen Einsatzes R in der jeweiligen Durchgangsöffnung A5 bzw. A6 jeweils auslenken kann. Auch hier verfügen die Kegelmantelflächen jeweils über je einen Öffnungswinkel δ von 30° zur Unterseite G2.

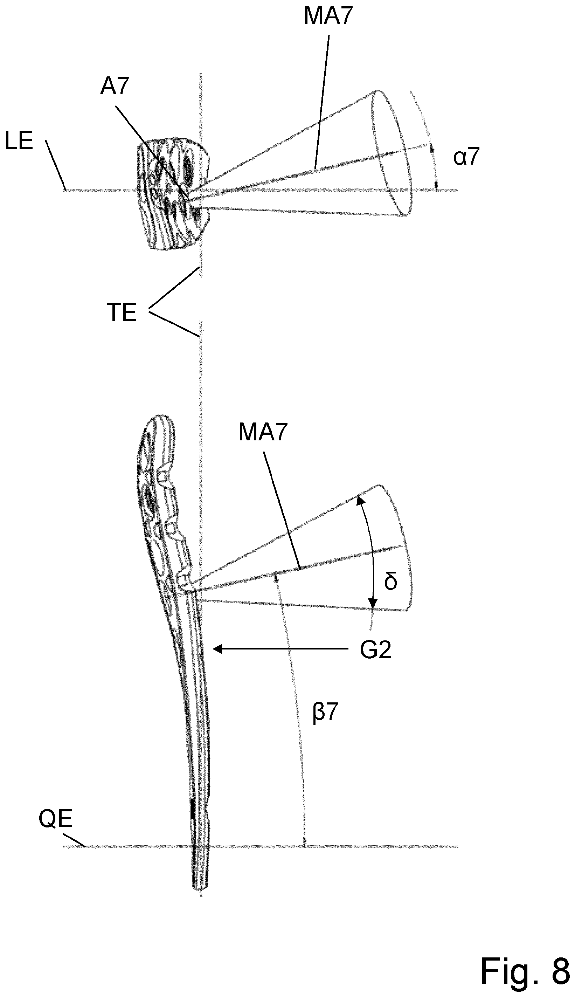

In Fig. 8 ist die Mittelachse MA7 der einzelnen Durchgangsöffnung A7 gezeigt, wobei dabei anhand der oberen Darstellung in Fig. 8 zu erkennen ist, dass die Mittelachse MA7 in Längsrichtung des Grundkörpers G betrachtet gegenüber der Längsmittelebene LE zu der posterioren Seite PS hin um einen Winkel α7 verkippt ist. Zudem verläuft die Mittelachse MA7 der Durchgangsöffnung A7 in Längsrichtung des Grundkörpers G betrachtet zumindest weitestgehend orthogonal zu dem dortigen Bereich des Kopfabschnitts KA. Aus der unteren Darstellung in Fig. 8 ist zudem ersichtlich, dass die Mittelachse MA7 der Durchgangsöffnung A7 in Querrichtung des Grundkörpers G betrachtet gegenüber der Querebene QE auf der Unterseite G2 proximal ansteigend unter einem Winkel β7 im Bereich 10° bis 16° verkippt ist. Erneut kann eine durch die Durchgangsöffnung A7 hindurchgeführte Schraube SK über den Einsatz R innerhalb einer Kegelmantelflächen ausgelenkt werden, wie in Fig. 8 angedeutet. Die Kegelmantelflächen verfügt dabei über einen Öffnungswinkel δ von 30° zur Unterseite G2.

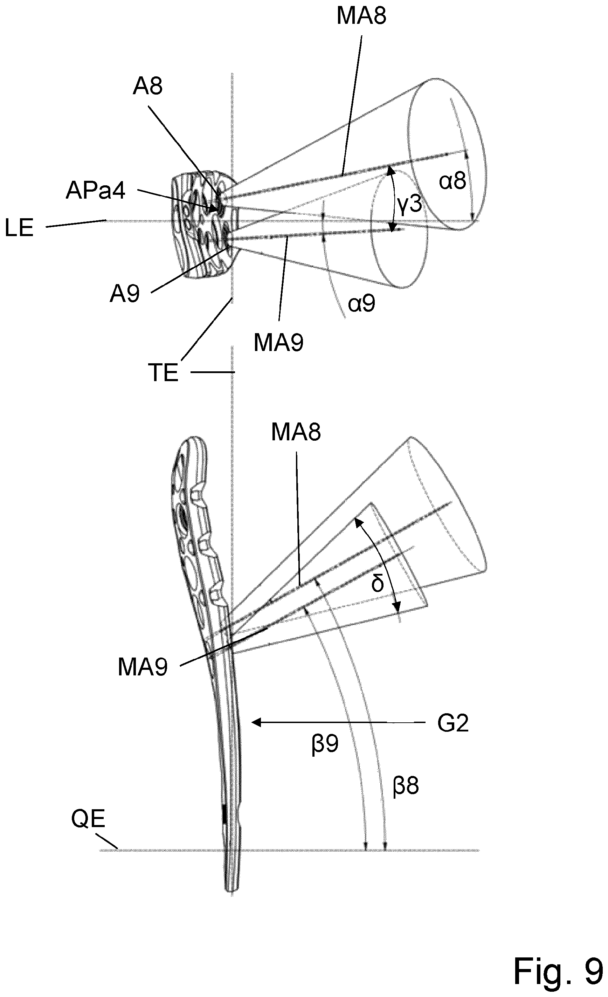

Aus Fig. 9 sind Mittelachsen MA8 und MA9 der Durchgangsöffnungen A8 und A9 des vierten Durchgangsöffnungspaares APa4 ersichtlich. Dabei ist zu erkennen, dass die beiden Mittelachsen MA8 und MA9 der beiden Durchgangsöffnungen A5 und A6 in Längsrichtung betrachtet (obere Darstellung in Fig. 9) auf der Unterseite G2 divergierend zueinander verlaufen, wobei dies dabei mit einem Winkel γ3 vorgenommen ist. Die Mittelachse MA8 der Durchgangsöffnung A8 ist gegenüber der Längsmittelebene LE unter einem Winkel α8 zu der posterioren Seite PS verkippt. Die Mittelachse MA9 der Durchgangsöffnung A9 ist gegenüber der Längsmittelebene LE unter einem Winkel α9 zu der posterioren Seite PS verkippt.

Zudem sind die beiden Mittelachsen MA8 und MA9 auf der Unterseite G2 des Grundkörpers G in Querrichtung des Grundkörpers G betrachtet (untere Darstellung in Fig. 9) jeweils proximal ansteigend verkippt. Hierbei sind die Mittelachse MA8 der Durchgangsöffnung A8 und die Mittelachse A9 der Durchgangsöffnung A9 gegenüber der Querebene QE unter einem Winkel β8, β9 im Bereich 20° bis 35° proximal verkippt. Erneut sind in Fig. 9 Kegelmantelflächen angedeutet, innerhalb welchen sich die jeweilige Schraube SK aufgrund des jeweiligen Einsatzes R in der jeweiligen Durchgangsöffnung A8 bzw. A9 auslenken kann. Auch hier verfügen die Kegelmantelflächen jeweils über je einen Öffnungswinkel δ von 30° zur Unterseite G2.

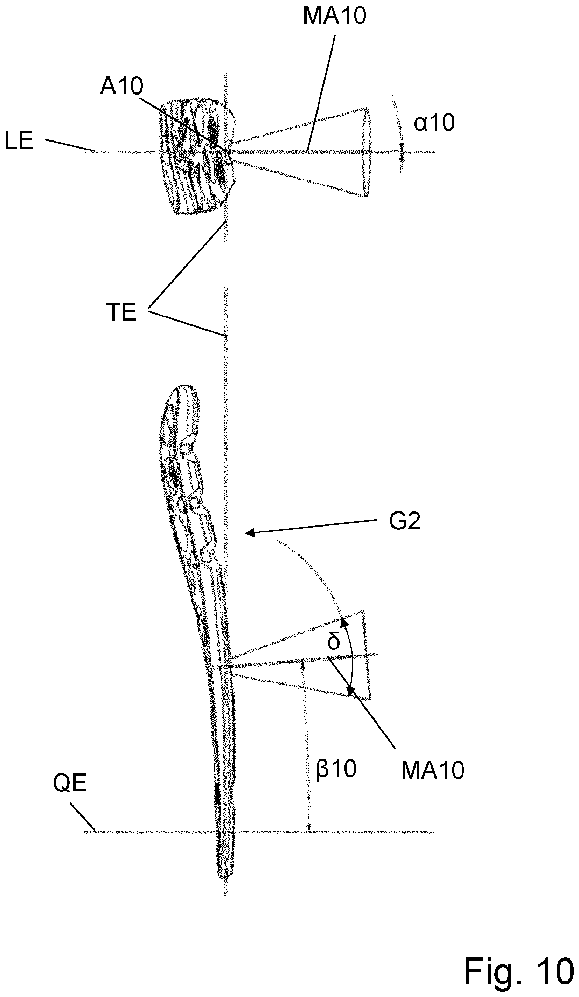

Ferner zeigt Fig. 10 die Mittelachse MA10 der Durchgangsöffnung A10 des Schaftabschnitts SA. Dabei ist zu erkennen, dass die Mittelachse MA10 in Längsrichtung des Grundkörpers G betrachtet (obere Darstellung in Fig. 10) gegenüber der Längsmittelebene LE um einen Winkel α10 zu der posterioren Seite PS hin, verkippt ist. Des Weiteren ist die Mittelachse MA10 auf der Unterseite G2 des Grundkörpers G in Querrichtung des Grundkörpers G betrachtet (untere Darstellung in Fig. 5) proximal ansteigend unter einem Winkel β10 im Bereich 1° bis 8°, verkippt. Neben einer möglichen Verschiebung der jeweils aufgenommenen Schraube SK in Längsrichtung entlang der als Langloch LL gestalteten Durchgangsöffnung A10 kann die jeweilige Schraube SK zudem in der Durchgangsöffnung A10 innerhalb einer Kegelmantelfläche mit einem Öffnungswinkel δ von 30° zur Unterseite G2 ausgelenkt werden.

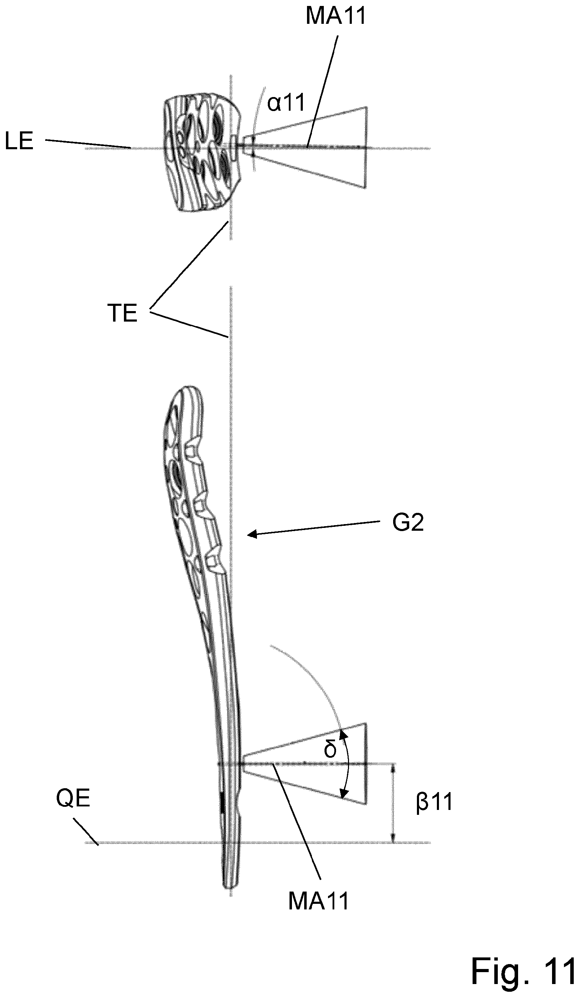

In Fig. 11 ist die Mittelachse MA11 der Durchgangsöffnung A11 des Schaftabschnitts SA gezeigt, wobei dabei anhand der oberen Darstellung in Fig. 11 zu erkennen ist, dass die Mittelachse MA11 in Längsrichtung des Grundkörpers G betrachtet gegenüber der Längsmittelebene LE um einen Winkel α11 zu der anterioren Seite AS hin verkippt ist. Zudem verläuft die Mittelachse MA11 der Durchgangsöffnung A11 gegenüber der Querebene QE auf der Unterseite G2 um einen Winkel β11 proximal verkippt (untere Darstellung von Fig. 11). Erneut kann eine durch die Durchgangsöffnung A7 hindurchgeführte Schraube SK über den Einsatz R innerhalb einer Kegelmantelflächen ausgelenkt werden, was auch in Fig. 11 angedeutet ist. Die Kegelmantelflächen verfügt dabei über einen Öffnungswinkel δ von 30° zur Unterseite G2.

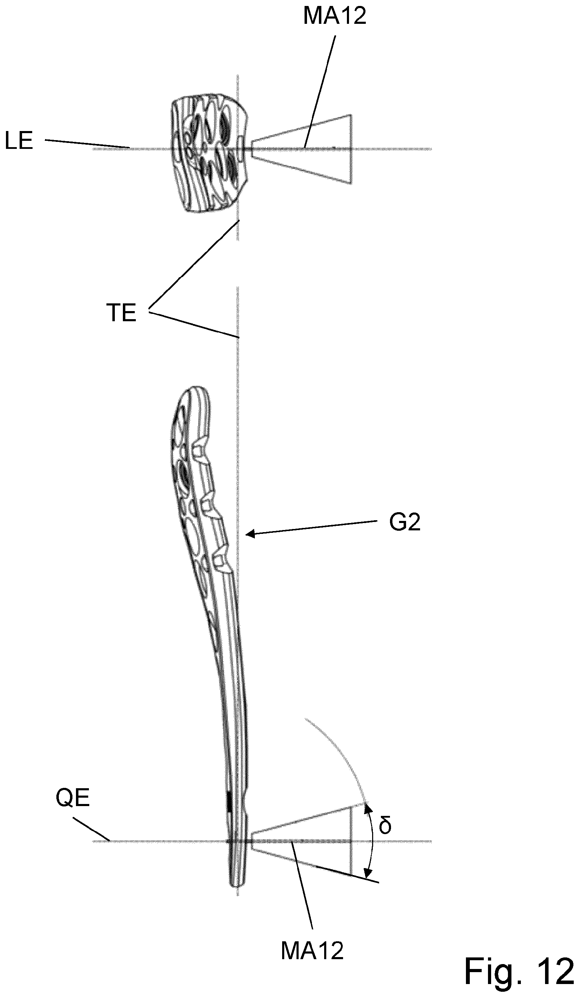

Schließlich zeigt noch Fig. 12 die Mittelachse MA12 der Durchgangsöffnung A12 des Schaftabschnitts SA. Diese Mittelachse MA der12 Durchgangsöffnung A12 ist dabei sowohl zu der Längsmittelebene LTE, als auch der Querebene QE jeweils kongruent, da die Ebenen LE, QE und auch TE jeweils auf diese Durchgangsöffnung A12 bezogen sind. Erneut kann eine durch die Durchgangsöffnung A12 hindurchgeführte Schraube SK über den eingefügten Einsatz R innerhalb einer Kegelmantelflächen ausgelenkt werden, wie in Fig. 12 angedeutet ist. Die Kegelmantelflächen verfügt dabei über einen Öffnungswinkel δ von 30° zur Unterseite G2.

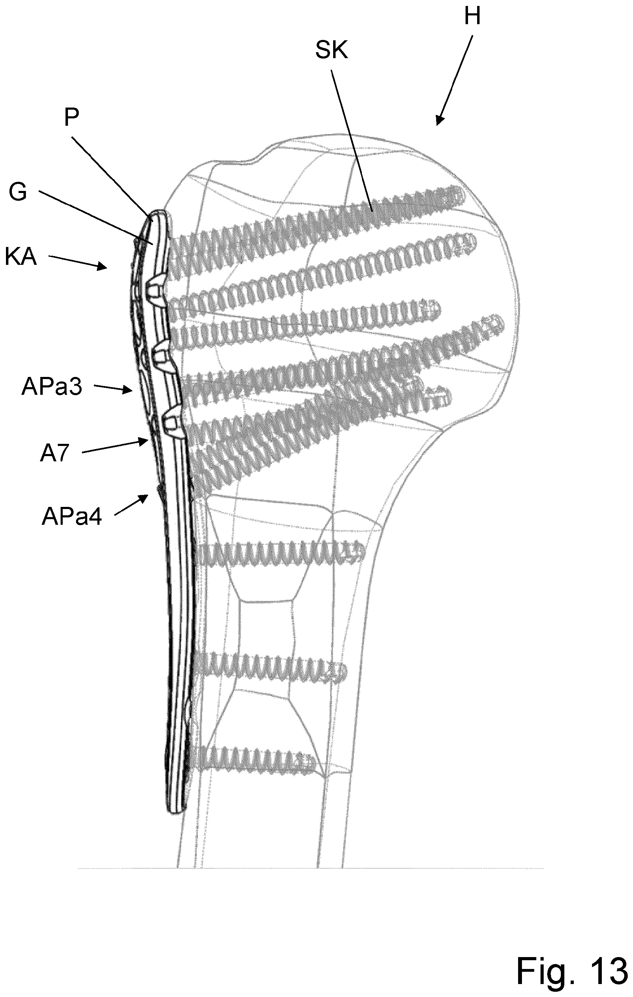

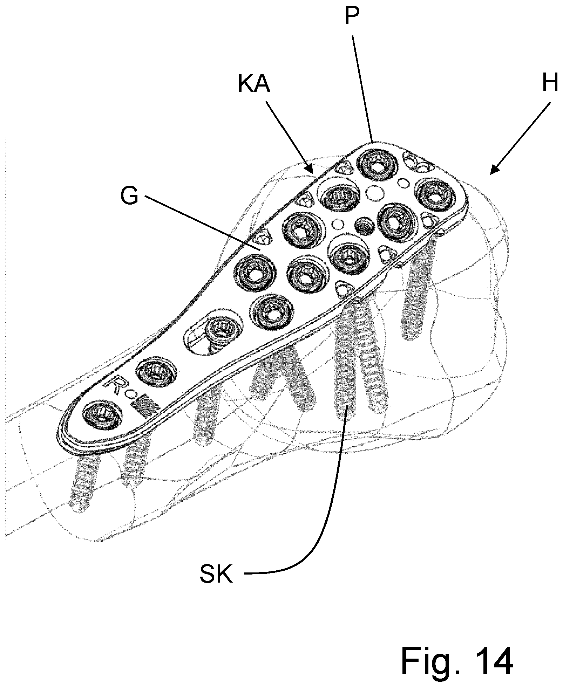

In den Fig. 13 und 14 ist die erfindungsgemäße Osteosyntheseplatte P jeweils im befestigten Zustand am proximalen Ende eines rechten Humerus H gezeigt. Wie zu erkennen ist, liegt die Osteosyntheseplatte P dabei an der Unterseite G2 ihres Grundkörpers G mit dem Kopfabschnitt KA im Bereich des Kopfs und Halses des Humerus H auf, während der Schaftabschnitt SA des Grundkörpers G am Schaft des Humerus H aufliegt. Durch die Durchgangsöffnungen A1 bis A12 sind dabei jeweils Schrauben SK hindurchgeführt, um sowohl den Kopfabschnitt KA als auch den Schaftabschnitt SA jeweils mit dem proximalen Humerus H zu verbinden und dadurch Knochenfragmente zueinander auszurichten und zu stabilisieren, die aufgrund einer proximalen Humerusfraktur gebildet sind. In den Fig. 13 und 14 ist zudem hinsichtlich der Befestigung des Kopfabschnitts KA die Auffächerung der hindurchgeführten Schrauben SK zu erkennen, durch welche eine zuverlässige Befestigung des Kopfabschnitt KA sichergestellt ist. Dabei ist auch insbesondere aus Fig. 13 erkennbar, dass die durch das vierte Durchgangsöffnungspaar APa4 hindurchgeführten Schrauben SK aufgrund der stärkeren proximalen Verkippung der Mittelachsen MA8 und MA9 sich auf Einschraubtiefe der zugehörigen Schrauben SK proximal bis in den Bereich der durch die Durchgangsöffnung A7 und der durch das dritte Durchgangsöffnungspaar APa3 hindurchgeführten Schrauben SK erstrecken.

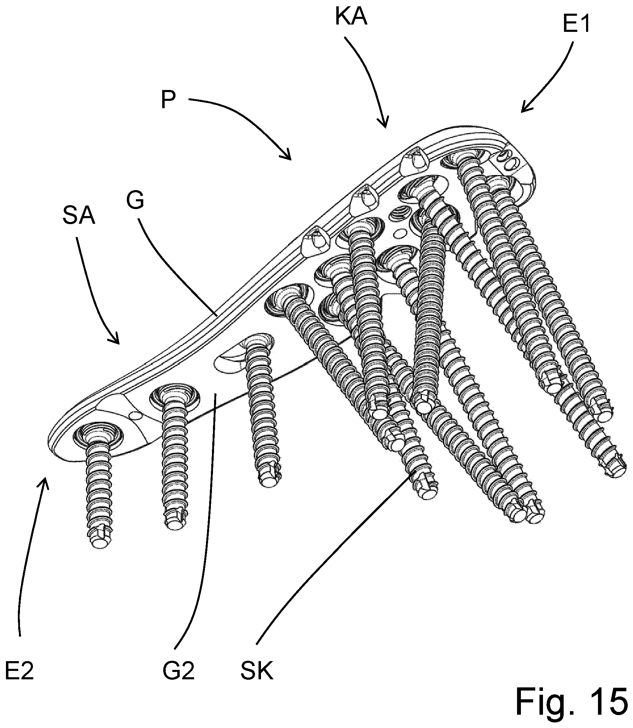

Fig. 15 zeigt eine Ansicht der Unterseite G2 der Osteosyntheseplatte P mit darin eingefügten Schrauben SK. Aus dieser Ansicht geht die Auffächerung im Bereich des Kopfabschnitts deutlich hervor. Selbstverständlich muss bei der Anwendung der Osteosyntheseplatte P nicht in jede der Durchgangsöffnungen A1 bis A12 eine Schraube SK eingeführt werden. Der Operateur kann abhängig von der individuellen Fraktur nur in ausgewählten Durchgangsöffnungen A1 bis A12 eine Schraube SK einführen, um die Osteosyntheseplatte P am Humerus H zu befestigen.

### Bezugszeichenliste

- P: Osteosyntheseplatte
- G: Grundkörper
- G1: Oberseite
- G2: Unterseite
- KA: Kopfabschnitt
- SA: Schaftabschnitt
- E1: proximales Ende
- E2: distales Ende
- LE: Längsmittelebene
- QE: Querebene
- TE: Tiefenebene
- AS: anteriore Seite
- PS: posteriore Seite
- K: Kante
- N: Nahtanker
- A1 bis A12: Durchgangsöffnungen
- SK: Schrauben
- APa1: erstes Durchgangsöffnungspaar
- APa2: zweites Durchgangsöffnungspaar
- APa3: drittes Durchgangsöffnungspaar
- APa4: viertes Durchgangsöffnungspaar
- R: Einsatz
- LL: Langloch
- BA: Bohrung
- MA1 bis MA12: Mittelachsen
- α1 bis α11: Winkel
- β1 bis β11: Winkel
- γ1 bis γ3: Winkel
- δ: Öffnungswinkel
- H: Humerus

## Patentansprüche

1. Osteosyntheseplatte (P), welche für eine Befestigung am proximalen Humerus (H) zur Fixierung von Knochenfragmenten vorgesehen ist,
- wobei die Osteosyntheseplatte (P) einen länglichen Grundkörper (G) mit einer Oberseite (G1) und mit einer der Oberseite (G1) abgewandt liegenden Unterseite (G2) aufweist sowie über einen Kopfabschnitt (KA) und einen Schaftabschnitt (SA) verfügt,
- wobei die Unterseite (G2) zur Auflage der Osteosyntheseplatte (P) auf den Knochenfragmenten eingerichtet ist,
- wobei an dem Kopfabschnitt (KA) und an dem Schaftabschnitt (SA) jeweils mehrere Durchgangsöffnungen (A1 bis A12) ausgestaltet sind, die für die Aufnahme von je einer Schraube (SK) vorgesehen sind und jeweils zwischen der Oberseite (G1) und der Unterseite (G2) des Grundkörpers (G) entlang je einer zugeordneten Mittelachse (MA1 bis MA12) verlaufen,
- wobei an dem Kopfabschnitt (KA) vier Durchgangsöffnungspaare (APa1, APa2, APa3, APa4) sowie eine einzelne Durchgangsöffnung (A7) ausgestaltet sind,
- wobei am Kopfabschnitt (K) ausgehend von einem proximalen Ende (E1) in Längsrichtung des Grundkörpers (G) zunächst das erste Durchgangsöffnungspaar (APa1), dann das zweite Durchgangsöffnungspaar (APa2), dann das dritte Durchgangsöffnungspaar (APa3), dann die einzelne Durchgangsöffnung (A7) und schließlich das vierte Durchgangsöffnungspaar (APa4) aufeinander folgen,
- und wobei die Mittelachsen (MA1, MA2) der Durchgangsöffnungen (A1, A2) des ersten Durchgangsöffnungspaares (APa1) in Längsrichtung des Grundkörpers (G) betrachtet zumindest weitestgehend parallel zueinander verlaufen,
**dadurch gekennzeichnet,**
- **dass** die Mittelachsen (MA3, MA4) der Durchgangsöffnungen (A3, A4) des zweiten Durchgangsöffnungspaares (APa2) in Längsrichtung des Grundkörpers (G) betrachtet auf der Unterseite (G2) kreuzend zueinander verlaufen,
- **dass** die Mittelachsen (MA5, MA6) der Durchgangsöffnungen (A5, A6) des dritten Durchgangsöffnungspaares (APa3) in Längsrichtung des Grundkörpers (G) betrachtet auf der Unterseite (G2) divergierend zueinander verlaufen,
- und **dass** die Mittelachsen (MA8, MA9) der Durchgangsöffnungen (A8, A9) des vierten Durchgangsöffnungspaares (APa4) in Längsrichtung des Grundkörpers (G) betrachtet zumindest weitestgehend parallel zueinander verlaufen.

2. Osteosyntheseplatte (P) nach Anspruch 1, **dadurch gekennzeichnet, dass** eine am proximalen Ende (E1) liegende Kante (K) des Kopfabschnitts (KA) zu einer Seite des Grundkörpers (G), bevorzugt zu einer posterioren Seite (PS) des Grundkörpers (G), hin abfällt, wobei die Kante (K) gegenüber einer Querebene (QE) des Grundkörpers (G) bevorzugt um 10° bis 20°, besonders bevorzugt um 13 bis 17° sowie besonders bevorzugt um 15° abfällt.

3. Osteosyntheseplatte (P) nach Anspruch 1 oder Anspruch 2, **dadurch gekennzeichnet, dass** die jeweils zugehörigen Durchgangsöffnungen (A1, A2, A3, A4, A5, A6, A8, A9) des jeweiligen Durchgangsöffnungspaares (APa1, APa2, APa3, APa4) in Längsrichtung des Grundkörpers (G) versetzt zueinander angeordnet sind.

4. Osteosyntheseplatte (P) nach Anspruch 3, **dadurch gekennzeichnet, dass** bei dem ersten Durchgangsöffnungspaar (APa1), dem zweiten Durchgangsöffnungspaar (APa2) und dem dritten Durchgangsöffnungspaar (APa3) die jeweils auf einer posterioren Seite (PS) des Grundkörpers (G) liegende Durchgangsöffnung (A1; A3; A5) in Längsrichtung distal versetzt zu der jeweils auf einer anterioren Seite (AS) des Grundkörpers (G) liegenden Durchgangsöffnung (A2; A4; A6) angeordnet ist.

5. Osteosyntheseplatte (P) nach Anspruch 3 oder 4, **dadurch gekennzeichnet, dass** bei dem vierten Durchgangsöffnungspaar (APa4) die auf einer posterioren Seite (PS) des Grundkörpers (G) liegende Durchgangsöffnung (A8) in Längsrichtung proximal versetzt zu der jeweils auf einer anterioren Seite (AS) des Grundkörpers (G) liegenden Durchgangsöffnung (A9) angeordnet ist.

6. Osteosyntheseplatte (P) nach einem der vorhergehenden Ansprüche, **dadurch gekennzeichnet, dass** die Mittelachse (MA7) der einzelnen Durchgangsöffnung (A7) des Kopfabschnitts (KA) in Längsrichtung des Grundkörpers (G) betrachtet zumindest weitestgehend orthogonal zu dem dortigen Bereich des Kopfabschnitts (KA) verläuft.

7. Osteosyntheseplatte (P) nach einem der vorhergehenden Ansprüche, **dadurch gekennzeichnet, dass** der Kopfabschnitt (KA) in Längsrichtung des Grundkörpers (G) betrachtet gegenüber dem Schaftabschnitt (SA) zu einer anterioren Seite (AS) des Grundkörpers (G) hin geneigt verdreht ist, wobei die Verdrehung bevorzugt um 4° bis 10° vorgenommen ist.

8. Osteosyntheseplatte (P) nach einem der vorhergehenden Ansprüche, **dadurch gekennzeichnet, dass** die Mittelachsen (MA1, MA2) der Durchgangsöffnungen (A1, A2) des ersten Durchgangsöffnungspaares (APa1), die Mittelachsen (MA3, MA4) der Durchgangsöffnungen (A3, A4) des zweiten Durchgangsöffnungspaares (APa2), die Mittelachsen (MA5, MA6) der Durchgangsöffnungen (A5, A6) des dritten Durchgangsöffnungspaares (APa3), die Mittelachse (MA7) der einzelnen Durchgangsöffnung (A7) sowie die Mittelachsen (MA8, MA9) der Durchgangsöffnungen (A8, A9) des vierten Durchgangsöffnungspaares (APa4) auf der Unterseite (G2) jeweils in Richtung des proximalen Endes (E1) verkippt sind.

9. Osteosyntheseplatte (P) nach einem der vorangehenden Ansprüche, **dadurch gekennzeichnet, dass** die Mittelachsen (MA8, MA9) der Durchgangsöffnungen (A8, A9) des vierten Durchgangsöffnungspaares (APa4) in Querrichtung des Grundkörpers (G) betrachtet die Mittelachsen (MA7, MA5, MA6) der einzelnen Durchgangsöffnung (A7) und der Durchgangsöffnungen (A5, A6) des dritten Durchgangsöffnungspaares (APa3) auf Einschraubtiefe der zugehörigen Schrauben (SK) schneidend verlaufen.

10. Osteosyntheseplatte (P) nach einem der vorhergehenden Ansprüche, **dadurch gekennzeichnet, dass** der Schaftabschnitt (SA) zumindest weitestgehend eben verläuft und eine Längsmittelebene (LE) des Grundkörpers (G) und eine orthogonal zu der Längsmittelebene (LE) verlaufende Querebene (QE) des Grundkörpers (G) definiert.

11. Osteosyntheseplatte (P) nach Anspruch 10, **dadurch gekennzeichnet,**
- **dass** die Mittelachsen (MA1, MA2) der Durchgangsöffnungen (A1, A2) des ersten Durchgangsöffnungspaares (APa1) gegenüber der Querebene (QE) auf der Unterseite (G2) in Richtung des proximalen Endes (E1) in einem Winkel (β1, β2) im Bereich 10° bis 25° ausgerichtet sind, und/oder
- **dass** die Mittelachsen (MA3, MA4) der Durchgangsöffnungen (A3, A4) des zweiten Durchgangsöffnungspaares (APa2) in Längsrichtung des Grundkörpers (G) betrachtet auf der Unterseite (G2) unter einem Winkel (γ1) im Bereich 40° bis 53° kreuzend ausgerichtet sind, und/oder
- **dass** die Mittelachsen (MA3, MA4) der Durchgangsöffnungen (A3, A4) des zweiten Durchgangsöffnungspaares (APa2) gegenüber der Querebene (QE) auf der Unterseite (G2) in Richtung des proximalen Endes (E1) in einem Winkel (β3, β4) im Bereich 5° bis 20° ausgerichtet sind, und/oder
- **dass** die Mittelachsen (MA5, MA6) der Durchgangsöffnungen (A5, A6) des dritten Durchgangsöffnungspaares (APa3) in Längsrichtung des Grundkörpers (G) betrachtet auf der Unterseite (G2) unter einem Winkel (γ2) im Bereich 15° bis 30° divergierend ausgerichtet sind, und/oder
- **dass** die Mittelachsen (MA5, MA6) der Durchgangsöffnungen (A5, A6) des dritten Durchgangsöffnungspaares (APa3) gegenüber der Querebene (QE) auf der Unterseite (G2) in Richtung des proximalen Endes (E1) in einem Winkel (β5, β6) im Bereich 5° bis 16° ausgerichtet sind, und/oder
- **dass** die Mittelachse (MA7) der einzelnen Durchgangsöffnung (A7) gegenüber der Querebene (QE) auf der Unterseite (G2) in Richtung des proximalen Endes (E1) in einem Winkel (β7) im Bereich 10° bis 16° ausgerichtet ist, und/oder
- **dass** die Mittelachsen (MA8, MA9) der Durchgangsöffnungen (A8, A9) des vierten Durchgangsöffnungspaares (APa4) in Richtung des proximalen Endes (E1) in einem Winkel (β8, β9) im Bereich 20° bis 35° ausgerichtet sind.

12. Osteosyntheseplatte (P) nach einem der vorhergehenden Ansprüche, **dadurch gekennzeichnet, dass** an dem Schaftabschnitt (SA) eine erste Durchgangsöffnung (A10), eine zweite Durchgangsöffnung (A11) und eine dritte Durchgangsöffnung (A12) ausgestaltet sind, die in Längsrichtung des Schaftabschnitts (SA) hintereinanderliegend sowie jeweils eine Längsmittelebene (LE) des Grundkörpers (G) schneidend angeordnet sind.

13. Osteosyntheseplatte (P) nach Anspruch 12, **dadurch gekennzeichnet, dass** die benachbart zu dem Kopfabschnitt (KA) liegende, erste Durchgangsöffnung (A10) als Langloch (LL) ausgeführt ist.

14. Osteosyntheseplatte (P) nach einem der vorhergehenden Ansprüche, **dadurch gekennzeichnet, dass** eine oder mehrere der Durchgangsöffnungen (A1 bis A12) jeweils ein Verschwenken der zugeordneten Schraube (SK) gegenüber der jeweiligen Mittelachse (MA1 bis MA12) zulassen, bevorzugt im Bereich einer Kegelmantelfläche, besonders bevorzugt einer Kegelmantelfläche mit einem Öffnungswinkel (δ) von 30°.

15. Osteosyntheseplatte (P) nach Anspruch 14, **dadurch gekennzeichnet, dass** die jeweilige das Verschwenken zulassende Durchgangsöffnung (A1 bis A9, A11, A12) mit einem Einsatz (R) versehen ist.
